Europäisches Patentamt

European Patent Office

Office européen des brevets

(11) Publication number: **0 080 379**
**B1**

## (12) EUROPEAN PATENT SPECIFICATION

(45) Date of publication of patent specification: **26.03.86**

(21) Application number: **82306262.5**

(22) Date of filing: **24.11.82**

(51) Int. Cl.⁴: **A 61 M 25/00, F 16 L 37/28**

(54) Connector assembly for liquid flow systems.

(30) Priority: **25.11.81 GB 8135609**

(43) Date of publication of application:
**01.06.83 Bulletin 83/22**

(45) Publication of the grant of the patent:
**26.03.86 Bulletin 86/13**

(84) Designated Contracting States:
**AT BE CH DE FR GB IT LI NL SE**

(56) References cited:
**EP-A-0 038 470**
**WO-A-80/01507**
**CH-A- 337 373**
**DE-A-1 950 263**
**DE-A-2 830 800**
**GB-A-1 192 986**
**US-A-3 417 750**
**US-A-3 642 037**

(73) Proprietor: **SCANDINAVIAN MEDICAL SUPPLY LIMITED**
**2 Hyde Park Place**
**London W2 2LH (GB)**

(72) Inventor: **Tranberg, Poul**
**Markedsvej 6**
**DK-6870 Olgod (DK)**

(74) Representative: **Warren, Anthony Robert et al**
**BARON & WARREN 18 South End Kensington**
**London W8 5BU (GB)**

Courier Press, Leamington Spa, England.

## Description

This invention relates to a plug-and-socket type connector assembly incorporating a cut-off valve which is intended to be substantially drip-proof in operation, the connector assembly being primarily intended to be incorporated between a surgical drain of a patient and a liquid receptacle such as a plastics bag.

Various self-sealing connections between a drainage hose and a liquid receptacle are known, but with prior constructions liquid spillage tends to occur during its process of disconnecting the drainage hose from the receptacle, which process, in some cases, must take place several times per day. Since, in surgical drainage, the said liquid is often urine, blood or other liquid, containing pus, it will be evident that such spillage will be considered as hazardous, whether it occurs when a patient is in hospital or in private care.

Whilst International Application No. WO80/0157, United States Patent No. 3,642,037 and Swiss Patent No. 337,373 disclose connections which claim to avoid such spillage, these connectors are relatively complicated, and expensive to manufacture.

It is an object of the present invention to provide a self-sealing plug-and-socket type connector assembly incorporating a cut-off valve arrangement which is not only capable of effectively eliminating spillage or dripping during connection and disconnection, but is also capable of being manufactured relatively simply and inexpensively.

The present invention is directed to a connector assembly for use in a surgical liquid flow system of the type disclosed in Swiss Patent No. 337373 which includes releasably cooperable plug and socket parts, the plug part incorporating a first valve and the socket part incorporating a second valve, operable to permit liquid to flow between the parts when plugged together, and effectively to prevent liquid spillage from the parts when unplugged, both valves being actuated by the relative plugging and unplugging movement between the plug and socket parts, and plug part comprising a tubular plug body closed at one end and having at least one opening near the closed end, and the first valve of the plug part including a slide valve element comprising a sleeve surrounding the plug body, the plug body and sleeve being relatively slidable between a valve-closed position in which the sleeve closes the opening in the plug body, and a valve-open position in which the sleeve does not close the opening in the plug body, the socket part comprising a tubular socket body having an open end for receiving the sleeve and the closed end of the plug body, and the second valve of the socket part including a valve element, and a valve seat spaced from the open end of the socket body with which the valve element is sealingly cooperable, plugging movement between the plug and socket parts being operable both to cause displacement of the valve element, and of the plug body and sleeve, to their valve-open positions in which a liquid path is established between the plug and socket parts via the valve element and opening, and unplugging movement between the plug and socket parts being operable both to cause displacement of the valve element, and displacement of the plug body and sleeve, to their valve-closed positions, the sleeve including first locking means cooperable, in the valve-open position of the slide valve, with second locking means associated with the socket body to releasably lock the sleeve against separational movement relative to the socket part during initial unplugging movement of the plug body so as to cause the sleeve to slide relative to the plug body to its valve-closed position, the plug body, sleeve, socket body and valve element mutually cooperating, during unplugging movement, in a manner which effectively avoids any voids being formed therebetween capable of accommodating sufficient liquid to form drips.

The invention is characterised in that the sleeve is formed from a synthetic plastics material, in that the first locking means comprises resilient means formed integrally with the sleeve and cooperable with the second locking means to resiliently releasably lock the sleeve against said separational movement during said initial unplugging movement, and in that the valve element includes a generally transverse face cooperable with the valve seat.

The invention also consists in a surgical liquid flow system incorporating one or more disposable connector assemblies as above defined.

The liquid flow system may comprise a surgical drainage system including a liquid receptacle, a catheter or other drain means cooperable with a patient, and a liquid line, interconnecting the drain means and receptacle, incorporating a connector assembly as above defined.

In one embodiment, the components of the valve are integrated parts of the connecting members, i.e. the plug and socket parts, between the drainage line or hose and the receptacle or bag. The connecting action itself causes a valve to open in both the plug part and the socket part and thereby allows the passage of liquid through the system. Likewise, both valves are caused to close immediately the system is disconnected, avoiding waste from the connection or joint.

The plug part and socket part are, respectively, welded or glued to the ends of lengths or drainage hose leading to the bag and a catheter or other drain means. The tubular plug body, which is closed by an externally planar transverse end wall at its far or distal end, is provided with side openings in its cylindrical surface or wall quite near to the end wall. The tubular sleeve is slidable lengthwise between two extreme positions, namely between valve-open and valve-closed positions. This assembly can slide into the tubular socket body of the socket part to a fully introduced position of the sleeve, the sleeve and plug body moving in unison to this position. The plug body is then advanced a small distance further, causing the sleeve to slide back relatively to the plug body, to a position in which it clears the openings in the plug body and the openings communicate with the interior of the socket part. At the same time, the distal end of the plug body will lift valve element, for example disc valve, at the bottom or proximal end of the socket body and thus allow a liquid to flow through the system.

When, due to the need to exchange the bag, the plug part has to be disengaged from the socket part, the plug body, as it is being withdrawn, will initially lower the disc valve, allowing it to close, and at the same time cause the sleeve to slide forwards and cover the openings of the plug body. These movements will follow, surface to surface, and in no situation leave empty spaces or voids for liquid. It is of great importance, apart from the function of the valve arrangement described, to achieve an economic solution, as it must be remembered, that the article is to be considered as disposable.

In order that the invention may be more readily understood, reference will now be made to the accompanying drawings, in which:—

Figure 1 is a schematic fragmentary side view of part of a surgical drainage system incorporating a connector assembly embodying the invention, with the plug and socket parts of the assembly partially sectioned, and shown in their disconnected, valve-closed condition; and

Figure 2 is a view, similar to Figure 1, with the plug and socket parts shown in their interconnected, valve-open condition, on an enlarged scale.

The surgical drainage system includes a synthetic plastics bag 1 connected to a drainage line or hose 2a, 2b incorporating a coupling or connector assembly comprising a male or plug part 3 and a female or socket part 4.

The plug and socket parts are formed, for example moulded, from suitable resilient, self-supporting, synthetic plastics material or materials.

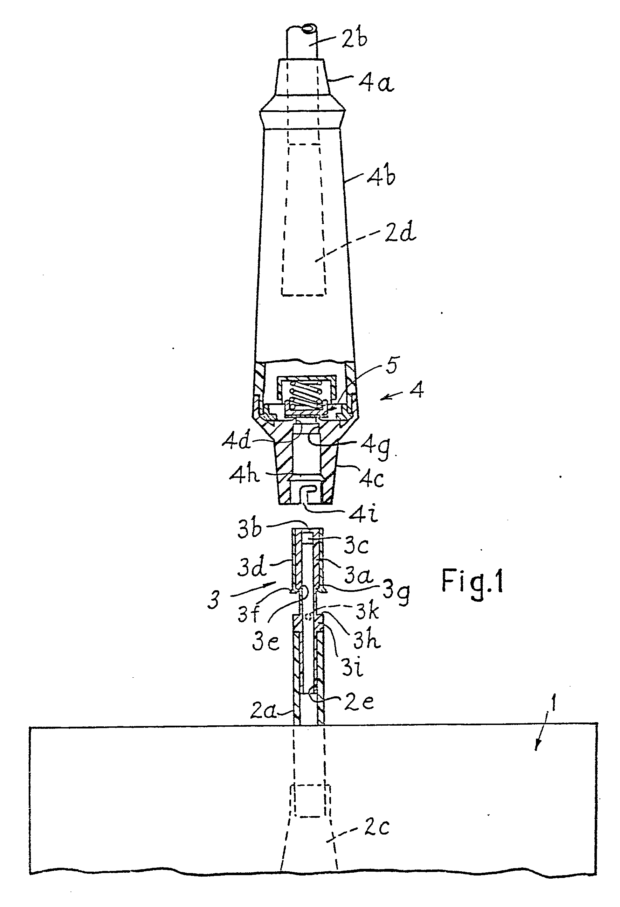

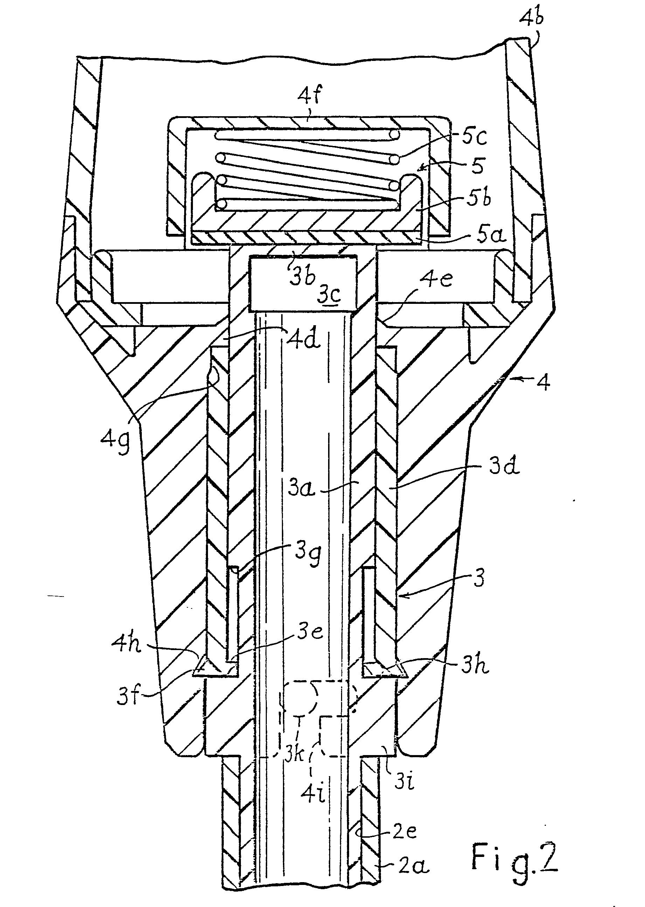

The plug part 3 includes a tubular, cylindrical body 3a, one end of which is secured at 2e, for example by welding or gluing, within an end of the section 2a of the drainage hose directly connected to the bag 1. The outer or distal end of the plug body 3a is closed by a transverse end wall 3b and, adjacent the end wall, the cylindrical side wall of the plug body is perforated by circumferentially extending openings 3c. A valve element comprising a cylindrical sleeve 3d closely fits the outer cylindrical surface of the plug body. The plug body is slidable axially within the sleeve 3d from a retracted or valve-closed position as shown in Figure 1, in which the outer or distal end of the sleeve is substantially flush with the planar outer surface of the transverse end wall 3b, and the sleeve overlies and seals off the openings 3c, to a valve-open position as shown in Figure 2, in which the distal end of the plug body 3a is advanced relative to the sleeve 3d to expose and open the openings 3c. The proximal end of the sleeve is provided with internal and external annular flanges 3e and 3f respectively. The internal flange 3e engages in a reduced diameter portion of the plug body, and is cooperable with stops formed by opposed shoulders 3g and 3h of the body to predetermine the range of sliding movement of the sleeve relative to the plug body. The shoulder 3h is formed at one end of an annular collar 3i integral with the plug body and formed with integral diametrically opposed pins 3k forming part of a bayonet-type locking arrangement.

The sleeve 3d is initially assembled to the plug body 3a by pressing the flanged proximal end of the sleeve over the distal end of the plug body, and to simplify this operation, the internal flange 3e is formed with a tapered radially inner surface to provide a lead-in.

The hose section 2a may terminate within the bag 1 in a non-return valve 2c (Fig. 2) which prevents liquid or gas within the bag from passing back up the hose.

The socket part 4 is also tubular, and has a reduced diameter end portion 4a secured, for example by welding or gluing, to an end of the section 2b of drainage hose intended to be connected to a catheter or other drain. The end portion 4a communicates with one end of an enlarged diameter cylindrical transparent portion 4b defining a tubular chamber, the other end of the chamber communicating with a tubular socket body 4c internally dimensioned to slidably receive the plug part 3. The cylindrical portion 4b is sufficiently resilient or flexible to permit it to be compressed manually, and the hose section 2b terminates, within the chamber, in a non-return valve 2d which prevents liquid or gas, and any bacteria etc., within the chamber from passing back up the hose section. The non-return valve 2d (and the valve 2c) may be of the type which comprises a length of open-ended, flat or collapsed flexible tubing, formed, for example, from two elongate flat strips of plastics material welded together along their longer edges.

The socket body 4c is provided, at its proximal end, with an internal annular flange 4d which serves as a stop for the sleeve 3d. The opening in the flange 4d is bounded by an annular ridge forming a seat 4e for a flap or disc valve element 5. The valve element 5 includes a circular, flat elastomeric sealing disc 5a bonded or otherwise secured to the flat end face of a closed-ended cylindrical support member 5b located within the cylindrical portion or chamber 4b. The disc valve element 5 is displaceable, and is biased by a coil spring 5c so as normally to urge the disc 5a towards the seat 4e to close, or tend to close, the opening in the flange 4d. The disc valve element 5 and bias spring 5c are located in a perforate housing 4f secured to the base of the cylindrical portion or chamber 4b, with the spring 5c maintained in compression between the disc valve element 5 and the underside of the top or end wall of the housing 4f. The inner diameter of the flange 4d, i.e. the diameter of the opening in the flange, is approximately the same as the external diameter of the distal end region of the plug body 3a, so that the latter end region will be snugly slidably received by the flange when the plug and socket parts are connected together as shown in Figure 2. The cylindrical passage within the socket body 4c is provided with a reduced diameter region 4g adjacent the flange 4d, which is slightly smaller in diameter than the distal end of the sleeve 3d. Between the region 4 and an annular recess 4h, the internal diameter of the socket body 4c approximates or is slightly greater than the diameter of the sleeve 3d but less than the diameter of the external flange 3f. The distal region of the passage in the socket body 4c, for example the region between the recess 4h and the open end of the body, may be internally stepped or tapered to provide an increased-diameter lead-in portion to facilitate insertion of the sleeve 3d. This distal region is also formed with a pair of slots 4i, forming part of the bayonet-type locking arrangement, and cooperable with the pins 3k.

The connector assembly operates as follows:—

In the disconnected condition of the plug and socket parts 3 and 4 shown in Figure 1, their associated valves are both closed. More specifically, the disc valve element 5 sealingly engages the valve seat 4e on the flange 4d, preventing liquid which flows from a patient, via a catheter connected to the hose section 2b, into the chamber in the cylindrical portion 4b, from flowing out of the socket body 4c. The distal end of the plug body 3a is retracted relative to the sleeve 3d, closing the openings 3c so as, after use and disconnection of the bag 1, to prevent flow of liquid from the hose section 2a connected to the bag. However, it is not essential for the sleeve 3d to close the openings 3c of a plug part of an unused bag prior to connection.

When the plug part 3, disposed as shown in Figure 1, is introduced into the socket body 4c, the frictional cooperation between the plug body 3a and sleeve 3d is preferably initially greater than that between the sleeve 3d and internal surface of the socket body 4c. Therefore, the plug body and sleeve travel together, i.e. in their valve-closed position (Figure 1), until the external flange 3f engages the distal end of the socket body 4c. Further insertion of the plug part then causes the plug body to advance relative to the sleeve until the proximal end of the sleeve engages the shoulder 3h. Still further insertion of the plug part causes the flange 3f to be flexed or compressed, and the plug body and sleeve advance in unison to the position shown in Figure 2, in which the flange 3f snaps into the recess 4h, and the closed distal end of the plug body passes through the flange 4d and lifts the disc valve element 5 from its seat 4e against the action of the spring 5c. Thus, liquid is free to flow through the socket part, through the perforate housing 4f, through the openings 3c, and into the plug part. This position is determined by the engagement and locking of the pins 3k in the slots 4i of the bayonet-type locking arrangement. Since, in this position, the sleeve 3d sealingly cooperates with the plug body 3a and socket body 4c, the distal end of the sleeve engages the flange 4d and sealingly cooperates with the reduced diameter region 4g, and the plug body is snugly received in the opening in the flange 4d, leakage between the plug and socket parts is prevented.

If, due to the friction between the sleeve and plug body 3a, the sleeve does not slide back on the plug body when, during insertion, the flange 3f engages the socket body, it will do so when its distal end encounters the region 4g. In any event, the sleeve will be positively driven fully home against the flange 4d, and the flange 3f will be snapped into the recess 4h, by engagement of the sleeve with the shoulder 3h.

Should the liquid flowing through the system tend to block the openings in the housing 4f and/or the openings 3c, for example due to coagulation, the flexible cylindrical socket portion 4b may be compressed, closing the non-return valve 2d, to pump or force the liquid through the openings and clear the blockage. The valve 2d isolates the patient from contaminants and pressure buildups downstream of the valve, and the valve 2c likewise prevents any contaminants, liquid, and pressure buildups in the bag from being transmitted to the connector assembly.

During withdrawal of the plug part from the socket part, the following procedure occurs. After the plug and socket parts have been relatively rotated to release the bayonet-type locking arrangement, the plug body 3a is firstly retracted relative to the socket body 4c and sleeve 3d until the upper surface of its transverse end wall 3b is flush with the upper edge of the valve seat 4e, so that the elastomeric disc 5a of the disc valve element 5 will seat and seal on the valve seat 4e without any significant voids, and therefore without any significant amounts of liquid being trapped, between the planar opposed surfaces of the disc 5a and distal end of the plug body. Withdrawal of the plug part alone continues since the sleeve is locked in its uppermost position by the engagement of its flange 3f in the recess 4h, until the plug part passes down through the opening in the flange 4d and the upper surface of its transverse end wall 3b is substantially flush with the upper or distal end of the sleeve 3d, so that the openings 3c are closed by the sleeve. In this condition, the flange 3e at the lower end of the sleeve 3d engages the shoulder 3g on the plug body, and as a result, further withdrawal of the plug body disengages the flange 3f from the recess 4h, and the sleeve and plug body (in their Figure 1 position) will be withdrawn in unison from the socket body until separated therefrom.

From the foregoing, it will be apparent that, whilst separational movement between the plug and socket parts is occurring, before the distal end of the plug body 3a is withdrawn from the opening in the flange 4d in the socket body 4c, the disc valve element 5 will follow the distal end of the plug body 3a down and rest on the valve seat 4e on the flange 4d, closing the opening therein, without at any time leaving any space between the disengaging members. Thus, separation is achieved without any dripping or other spillage or leakage.

According to a feature of the illustrated invention, the non-return valve 2b may, prior to assembly, be secured to a short length of pipe or hose, the latter being secured within the end portion 4a of the socket part. In this way, the valve 2b may be positively centrally located within the cylindrical portion or chamber 4b to ensure that the valve does not contact the cylindrical wall of the chamber which may otherwise cause the valve to malfunction, for example, remain partially open, even when the pressure in the chamber exceeds the pressure in the hose section 2b.

In order to prevent the transparent cylindrical portion 4b from becoming detached from the socket body 4c when the former is manually compressed, the socket body 4c may include a pair of concentric walls between which the lower end of the cylindrical portion 4b is mechanically located, and bonded or otherwise anchored in place. The cylindrical portion 4b may also be thickened adjacent its lower end to overlie the outer of the pair of concentric walls. The inner concentric wall may be provided by a flange on a separate re-entrant component, bonded into the socket body 4c, which also forms the housing 4f.

It is desirable to make the drainage hose for surgical drains from polythene since the hose will then be relatively flexible and soft, and therefore more comfortable to the patient. However, in prior drains, it is then necessary also to make the bag of polythene in order to enable the hose and bag to be readily bonded together. However, disposal of the polythene bags gives rise to problems since polythene, when incinerated, gives off corrosive or toxic by-products. In the illustrated embodiment, however, the hose section 2b constitutes the major proportion of the overall length of the drainage hose and can be formed from polythene, whereas the relatively short hose section 2a, and therefore the bag 1, can be formed from polyvinyl chloride. Polyvinyl chloride can not only be disposed of by incineration without the production of toxic or corrosive by-products, but it is also a relatively cheap material to employ, and its use enables the hose section and bag assembly to be manufactured at a much higher rate.

From the foregoing, it will be seen that there is provided a particularly advantageous form of effectively drip proof and contamination proof connector assembly. The connector assembly is relatively simple and fool-proof to manipulate, and is relatively simple and inexpensive to manufacture and assemble since it incorporates a minimum of individual components, all or the majority of which may be readily formed, for example moulded, from synthetic plastics material. It is economically viable for the connector assemblies to be treated as disposable items, avoiding the problems and expense of sterilization associated with re-use. The socket part and associated catheter may be retained connected to a patient, and used with a succession of plug parts and associated bags which are removed and disposed of when the bags are filled. When filled bags are unplugged, due to the effectively drip and void free valving arrangement, the risk of contamination being transmitted from or to the unplugged parts is effectively eliminated or significantly reduced.

It will be understood that various modifications may be made without departing from the scope of the present invention.

For example, the recess 4h in the socket body 4c could be replaced or supplemented by one or more ribs with which the sleeve flange 3f is releasably cooperable. Other means may be provided to ensure that, during and after removal of the plug part from the socket part, the sleeve is urged into, and/or indexed in, its closed position. Although, in the specific embodiment, the connector assembly is arranged with its plug part downstream of the socket part, the positions could be reversed. The disposition shown in the illustrated embodiment, however, has the advantage that the valve element 5 is assisted in closing by the normal flow of liquid through the drainage system, and the element is conveniently disposed in the chamber in the enlarged diameter socket portion 4b. The valve element 5 could, furthermore, take a form other than that of a disc valve, for example it could comprise a flexible flap valve secured or hinged at one side to the flange 4d.

Although, in the illustrated embodiment, connection and disconnection of the plug and socket parts is effected simply by a manual push or pull action respectively, accompanied by a twisting action to lock or unlock the bayonet-type locking arrangement, the locking arrangement could be omitted, or could take the form of a snap-acting connection, screw thread-type connection, or other type of releasable lock.

The shape, configurations and materials of the various components may be changed, and the connector assembly may be applied to liquid flow systems other than surgical drainage systems.

Although the plug and socket parts in the illustrated embodiment are permanently secured to the hose sections, they could alternatively be press-fitted or otherwise releasably attached to the hose sections or other fluid lines, etc. The plug and socket bodies could be formed, for example molded, integrally with the ends of their respective fluid lines, etc.

The diameter of the disc valve element 5 may be reduced to a value approaching that of the valve seat 4e, and may be prevented from lateral displacement relative to the valve seat to positions in which it may not fully cover the valve seat by means of upright rib-like guides within the housing 4f or on the element 5b. The spring 5c, at its upper end, may be located laterally, for example located in a recess in the top of the housing 4f.

The perforate housing 4f could be replaced by a rib or equivalent mounted in the chamber in the socket part, with which the spring 5c, a sponge-like pad, or equivalent resilient element, cooperates. Alternatively, the housing or rib, and resilient element, may be omitted, the disc or flap valve element relying on its own mass or resilience respectively, and/or the liquid pressure, to bias it to its closed position.

The range of sliding movement of the sleeve may be determined by means other than the sleeve flange 3e which cooperates, alternatively, with the shoulders 3g and 3h. For example, the shoulder 3h may be omitted, and instead, the flange 3e or equivalent may cooperate, with the distal end of the hose section 2a connected to the bag 1.

The flange 4d may be omitted, and the sleeve may be permitted to project through the opening in the valve seat in the socket body 4c. In this event, the fully inserted position of the plug body will be determined by the bayonet-type locking arrangement or other stop means. The distal end of the plug body may have an annular shoulder of the same diameter as that of the seat opening and sleeve, against the underside of which the sleeve abuts in its valve-closed position. The transverse upper surfaces of the end wall 3b and the latter annular shoulder will be effectively flat and co-planar to ensure that, during unplugging, no voids are formed between the latter surfaces and the underside of the valve element as it closes on its seat, to avoid formation of drips.

The drainage system may be employed with a hospitalised patient, although it could be used to advantage with a mobile patient as a urinary drain. In the latter case, since the likelihood of clots forming in the drained fluid is remote, it is not necessary to provide the pumping chamber 4b, and this may be omitted for reasons of space- and weight-saving, cost and convenience. The non-return valve 2d may be omitted, and the socket end portion 4a may be bonded directly to, or integrated with, the outer upper side wall of the socket body 4c.

In addition to, or as an alternative to, the bayonet-type locking arrangement, resilient cooperable snap-acting means may be provided to signify to a user when the plug and socket parts are fully engaged, and/or to index the sleeve in its valve-closed position during plugging or unplugging. For example the sleeve and/or plug body and/or socket body may be provided with annular ribs or other projections, and complementary cooperable grooves or depressions.

The system, for example the cylindrical socket portion 4b, may be provided with a vent or filter arrangement capable of venting gas (but not liquid) from the chamber in the event of a pressure build-up. Additionally or alternatively, such a vent or filter arrangement may be incorporated in the hose section 2b or in the connection for connecting the hose section to the catheter. The vent arrangement may also include a portion penetrable by a syringe needle or luer to permit injection into or extraction from the arrangement. One or both of the non-return valves 2c, 2d, may be omitted, or replaced by other types of valves.

**Claims**

1. A connector assembly for use in a surgical liquid flow system, including releasably cooperable plug and socket parts (3,4), the plug part (3) incorporating a first valve and the socket part incorporating a second valve, operable to permit liquid to flow between the parts when plugged together, and effectively to prevent liquid spillage from the parts when unplugged, both valves being actuated by the relative plugging and unplugging movement between the plug and socket parts, the plug part comprising a tubular plug body (3a) closed at one end (3b) and having at least one opening (3c) near the closed end, and the first valve of the plug part including a slide valve element comprising a sleeve (3d) surrounding the plug body, the plug body (3a) and sleeve (3d) being relatively slidable between a valve-closed position (Fig. 1) in which the sleeve closes the opening in the plug body, and a valve-open position (Fig. 2) in which the sleeve does not close the opening in the plug body, the socket part (4) comprising a tubular socket body (4c) having an open end for receiving the sleeve and the closed end of the plug body (3a), and the second valve of the socket part (4) including a valve element (5), and a valve seat (4e) spaced from the open end of the socket body with which the valve element (5) is sealingly cooperable, plugging movement between the plug and socket parts (3, 4) being operable both to cause displacement of the valve element (5), and of the plug body (3a) and sleeve (3d), to their valve-open positions in which a liquid path is established between the plug and socket parts via the valve element (5) and opening (3c), and unplugging movement between the plug and socket parts being operable both to cause displacement of the valve element (5), and displacement of the plug body (3a) and sleeve (3d), to their valve-closed positions, the sleeve (3d) including first locking means (3f) cooperable, in the valve-open position of the slide valve, with second locking means (4h) associated with the socket body (4c) to releasably lock the sleeve against separational movement relative to the socket part during initial unplugging movement of the plug body so as to cause the sleeve to slide relative to the plug body to its valve-closed position, the plug body, sleeve, socket body and valve element mutually cooperating, during unplugging movement, in a manner which effectively avoids any voids being formed therebetween capable of accommodating sufficient liquid to form drips, characterised in that the sleeve (3d) is formed from a synthetic plastics material, in that the first locking means (3f) comprises resilient means formed integrally with the sleeve and cooperable with the second locking means (4*h*) to resiliently releasably lock the sleeve (3*d*) against said separational movement during said initial unplugging movement, and in that the valve element (5) includes a generally transverse face cooperable with the valve seat (4*e*).

2. A connector assembly as claimed in claim 1, characterised in that the closed end of the plug body (3*a*) is closed by an externally generally planar transverse end wall (3*b*), and wherein the valve element (5) comprises a disc valve which, in the valve-closed position, has a generally planar transverse face presented towards the open end of the plug body-receiving open end of the socket body (4*c*), and sealingly cooperable with the valve seat (4*e*), the valve seat having an opening dimensioned to closely receive the end wall (3*b*), whereby, when in the valve-closed position at the transition between the valve-open and valve closed positions, the end wall (3*b*) cooperates closely with the disc valve (5) and the opening in the valve seat (4*e*) to prevent any significant liquid-accomodatable voids being formed therebetween.

3. A connector assembly as claimed in claim 1 or 2, characterised in that said first locking means (3*f*) comprises a resilient flange integral with the exterior of the sleeve (3*d*) at the end thereof remote from the opening in the plug body, the flange projecting towards the socket body and being cooperable with the second locking means (4*h*) which comprises a shoulder within the socket body (4*c*).

4. A connector assembly as claimed in claim 3, characterised in that said plug body (3*a*), socket body (4*c*) and sleeve (3*d*) are generally cylindrical, said flange (3*f*) is an annular flange, and said shoulder (4*h*) is defined by an annular recess within the socket body.

5. A connector assembly as claimed in claim 1, 2 or 3, characterised in that insertion of the plug part into the socket part during plugging movement urges the sleeve (3*d*) into abutment with the valve seat (4*e*), thereby to determine the maximum extent of insertion of the sleeve into the socket body, and to ensure that the plug body is advanced through the opening in the valve seat relative to the sleeve to a position in which the sleeve does not close the opening (3*c*) in the plug body, in which the end wall (3*b*) cooperates with the valve element (5) to disengage it from the valve seat (4*e*), and in which the opening (3*c*) in the plug body is located on the side of the valve seat (4*e*) remote from the sleeve.

6. A connector assembly as claimed in any preceding claim, characterised in that, during initial plugging movement, the sleeve (3*d*) frictionally grips the plug body (3*a*) with a force which is greater than that with which the socket body grips the sleeve, whereby the sleeve remains in a position on the plug body in which it closes the opening (3*c*) in the plug body during initial plugging movement.

7. A connector assembly as claimed in any preceding claim, characterised in that the internal cross-sectional dimension of the socket body is reduced in the region (4*g*) of the valve seat (4*e*), so as to increase the frictional grip of the socket body on the sleeve in said region (4*g*).

8. A connector assembly as claimed in claim 2, or any of claims 3 to 7 in combination with claim 2, characterised in that the generally planar transverse end wall (3*b*) of the plug body is generally co-planar with the end of the sleeve (3*d*) in the valve-closed position.

9. A surgical liquid flow system, or liquid flow apparatus, incorporating one or more disposable connector assemblies as claimed in any preceding claim, and comprising a surgical drainage system or apparatus including a liquid receptacle (1), a catheter or other drain means cooperable with a patient, and a liquid line (2), interconnecting the drain means and receptacle, incorporating the connector assembly.

10. The connector assembly, system or apparatus as claimed in any preceding claim, including at least one non-return valve (2*c* or 2*d*) to prevent flow in one direction through the connector assembly when in the valve-open position, and wherein the socket part (4) includes a flexible housing (4*b*) containing the second valve (5), the housing (4*b*) being manually compressible to force liquid through the connector assembly when in the valve-open position.

**Patentansprüche**

1. Eine Verbindungsvorrichtung für die Anwendung in einem Leitungssystem für chirurgische Flüssigkeiten, einschließlich vorübergehend ineinander greifender Stecker und Buchsenteile (3,4), der Steckerteil enthält ein erstes Ventil und der Buchsenteil enthält ein zweites Ventil, das im Betrieb eine Flüssigkeit zwischen den beiden zusammengesteckten Teilen durchfließen läßt und einen Flüssigkeitsverlust von den Teilen, wenn sie ausstöpselt sind, wirksam verhindert, beide Ventile werden betätigt durch die Bewegung des Zusammenstöpselns bzw. des Herausziehens von Stecker- und Buchsenteilen, der Steckerteil besteht aus einem rohrförmigen Steckerkörper (3a), an einem Ende (3b) mit mindestens einer Öffnung (3c), in der Nähe des geschlossenen Endes, und das erste Ventil des Steckerteiles hat ein Schubventilelement mit einer Muffe (3d), die den Steckerkörper umgibt, der Steckerkörper (3a) und die Muffe (3d), die verschiebbar ist zwischen einer geschlossenen Ventilstellung (Fig. 1), in der die Muffe die Öffnung im Steckerkörper verschließt und einer geöffneten Ventilstellung (Fig. 2), in der die Muffe die Öffnung im Steckerkörper nicht verschließt, der Buchsenteil (4) mit einem rohrförmigen Buchsenkörper (4c) hat ein offenes Ende, um die Muffe und das geschlossene Ende des Steckerkörpers (3a) aufzunehmen und das zweite Ventil des Buchsenteiles (4) umfaßt ein Ventilelement (5) und einen Ventilsitz (4d), etwas entfernt von dem offenen Ende des Buchsenkörpers, mit dem das Ventilelement (5) abdichtend zusammenwirkt, die Kupplungsbewegung zwischen den Stecker- und Buchsenteilen (3,4) bewirkt bei das eine Verschiebung des Ventilelementes (5) und des Steckerkörpers (3a) und der Muffe (3d) in die geöffnete Ventilstellung, wobei ein Flüssigkeitskanal zwischen den Stecker- und Buchsenteilen durch das Ventilelement (5) und die Öffnung (3c) hergestellt wird, und die Entkupplungsbewegung zwischen Stecker- und Buchsenteilen bewirkt eine Verschiebung des Ventilelementes (5) und Verschiebung des Steckerkörpers (3a) und der Muffe (3d) in die geschlossene Ventilstellung, die Muffe (3d) weist einer ersten Rastenarretierung (3f) auf, die, in der offenen Stellung des Schubventiles, mit der zweiten Rastenarretierung (4h) am Buchsenkörper (4c) zusammenwirkt, um die Muffe vorübergehend gegen eine Trennbewegung bezogen auf den Buchsenteil während der anfänglichen Entkupplungsbewegung des Steckerkörpers zu fixieren, sodaß die Muffe im Bezug auf den Steckerkörper in die geschlossene Ventilstellung gleitet, der Steckerkörper, die Muffe, der Buchsenkörper und das Ventilelement wirken während des Herausziehens wechselseitig zusammen und zwar so, daß jede Lücke, die dazwischen entsteht und genügend Flüssigkeit aufnehmen kann, um Tropfen zu bilden, vermieden wird, gekennzeichnet dadurch, daß die Muffe (3d) aus synthetischem Plastikmaterial geformt ist, das die erste Rastenarretierung (3f) ein federndes Material enthält, das mit der Muffe ein Ganzes bildet und in die zweite Rastenarretierung (4h) eingreift, um die Muffe (3d) gegen die Trennbewegung während des anfänglichen Herausziehens elastisch lösbar zu fixieren, und ferner, wobei daß das Ventilelement (5) im wesentlichen eine Querfläche einschließt, die in den Ventilsitz (4e) eingreift.

2. Verbindungsvorrichtung nach Anspruch 1, dadurch gekennzeichnet, daß das geschlossene Ende des Steckerkörpers (3a) durch eine äußere, im wesentlichen flachen quer liegenden Stirnwand (3b) geschlossen ist und worin das Ventilelement (5) ein Plattenventil umfaßt, das, in der geschlossenen Ventilstellung, eine im wesentlichen flache Querplatte hat, die gegen das offene Ende des den Buchsenkörper (4c) aufnehmenden offenen Endes des Steckerkörpers und abdichtend mit dem Ventilsitz (4e) zusammenwirkt, wobei der Ventilsitz eine Öffnung hat, die so dimensioniert ist, daß sie die Stirnwand (3b) genau aufnimmt, wodurch in der geschlossenen Ventilstellung beim Übergang von der geöffneten in die geschlossene Ventilstellung die Stirnwand (3b) genau mit dem Plattenventil (5) und der Öffnung im Ventilsitz (4e) zusammenpaßt, um jede bedeutende flüssigkeitsaufnehmende Lücken, die dazwischen gebildet werden, zu verhindern.

3. Verbindungsvorrichtung nach Anspruch 1 oder 2, dadurch gekennzeichnet, daß die erste Rastenarretierung (3f) einen federnden Flansch umfaßt, der mit dem Äußeren der Muffe (3d) an ihrem von der Öffnung im Steckerkörper entfernten Ende einstückig verbunden ist, wobei der Flansch gegen den Buchsenkörper vorspringt und mit der zweiten Rastenarretierung (4h), die eine Schulter innerhalb des Buchsenkörpers (4c) bildet, zusammenwirkt.

4. Verbindungsvorrichtung nach Anspruch 3, dadurch gekennzeichnet, daß der Steckerkörpers (3a), der Buchsenkörper (4c) und die Muffe (3d) im wesentlichen zylindrisch sind, deren Flansch (3f) ein ringförmiger Flansch ist und die Schulter (4h) als ringförmige Nut innerhalb des Buchsenkörpers ausgebildet ist.

5. Verbindungsvorrichtung nach Anspruch 1, 2 oder 3, dadurch gekennzeichnet, daß das Einführen des Steckerteiles in den Buchsenteil während des Zusammenkuppelns die Muffe (3d) gegen den Ventilsitz (4e) drückt, um dadurch das größte Ausmaß des Einführens der Muffe in den Buchsenkörper zu ermöglichen und zu gewährleisten, daß der Steckerkörper durch die Öffnung im Ventilsitz relativ zur Muffe vorgeschoben wird bis zu einer Stellung, in der die Muffe die Öffnung (3c) im Steckerkörper nicht verschließt, wobei die Stirnwand (3b) mit dem Ventilelement (5) zusammenwirkt, um es vom Ventilsitz (4e) zu lösen, und in der die Öffnung (3c) im Steckerkörper auf von der Muffe abgewandten Seite des Ventilsitzes (4e) angeordnet ist.

6. Verbindungsvorrichtung nach einem der vorhergegangenen Ansprüche, dadurch gekennzeichnet, daß die Muffe (3d) während der anfänglichen Kupplungsbewegung den Steckerkörper (3a) durch Reibung festhält und zwar mit einer Kraft, die größer ist als die, mit der der Buchsenteil die Muffe festhält, wodurch die Muffe auf dem Steckerkörper in einer Position bleibt, in der sie die Öffnung (3c) im Steckerkörper während der anfänglichen Kupplungsbewegung verschließt.

7. Verbindungsvorrichtung nach einem der vorhergegangenen Ansprüche dadurch gekennzeichnet, daß die innere Querabmeßung des Buchsenkörpers im Bereich (4g) des Ventilsitzes (4e) reduziert wird, um die Reibungsgriffigkeit des Buchsenkörpers auf die Muffe in diesem Bereich (4g) zu erhöhen.

8. Verbindungsvorrichtung nach Anspruch 2 oder einem der Ansprüche 3 bis 7 in Verbindung mit Anspruch 2, dadurch gekennzeichnet, daß die im wesentlichen flache querlaufende Stirnwand (3b) des Steckerkörpers in der geschlossenen Ventilstellung im wesentlichen in gleicher Ebene liegt mit der Stirnwand der Muffe (3d).

9. Ein chirurgisches Flüssigkeitsleitungssystem oder ein Gerät mit Flüssigkeitsleitungen, mit einer oder mehreren anwendbaren Verbindungsvorrichtungen nach einem der vorhergehenden Ansprüche, umfassend ein chirurgisches Drainagesystem oder ein Gerät mit einem Flüssigkeitsbehälter (1), einem Katheter oder einem anderen Drainagemittel, das an einen Patienten angeschlossen werden kann, und eine Flüssigkeitsleitung (2), die die Drainagemittel und den Behälter verbindet und die Verbindungsvorrichtung enthält.

10. Die Verbindungsvorrichtung, ein System oder ein Gerät nach einem der vorhergehenden Ansprüche, mit mindestens einem Rückschlagventil (2c oder 2d), das ein Fließen in eine Richtung durch die Verbindungsvorrichtung bei geöffneter Ventilstellung verhindert, und worin der Buchsenteil (4) ein flexibles Gehäuse (4b) umschließt, das das zweite Ventil (5) enthält, wobei das Gehäuse (4b) manuell zusammenpreßbar ist, um in der geöffneten Ventilstellung eine Flüssigkeit durch die Verbindung zu drücken.

## Revendications

1. Raccord à utiliser dans un système d'écoulement de liquide chirurgical comprenant une partie mâle et une partie femelle (3, 4) pouvant coopérer de manière détachable, la partie mâle (3) comprenant une première valve et la partie femelle une seconde valve propres à être actionnées pour permettre au liquide de s'écouler entre les deux parties lorsque celles-ci sont raccordées l'une à l'autre et pour empêcher efficacement tout épanchement de liquide des dites parties lorsque celles-ci sont débranchées, les deux valves étant actionnées par le mouvement relatif de branchement et de débranchement entre la partie mâle et la partie femelle, la partie mâle comprenant un corps mâle tubulaire (3a) fermé à une extrémité et présentant au moins une ouverture (3c) près de son extrémité fermée, et la première valve de la partie mâle comprenant un obturateur coulissant comprenant une douille (3d) entourant le corps mâle, le corps mâle (3a) et la douille (3d) pouvant coulisser l'un par rapport à l'autre entre une position de fermeture de la valve (Fig. 1) dans laquelle la douille obture l'orifice prévu dans le corps mâle et une position d'ouverture de la valve (Fig. 2) dans laquelle la douille n'obture pas l'orifice prévu dans le corps mâle, la partie femelle (4) comprenant un corps femelle tubulaire (4c) présentant une extrémité ouverte destinée à recevoir la douille et l'extrémité fermée du corps mâle (3a), et la seconde valve de la partie femelle (4) comprenant un obturateur (5) et un siège de valve (4e) espacé de l'extrémité ouverte du corps femelle avec laquelle l'obturateur (5) peut coopérer de manière étanche, le mouvement de branchement entre la partie mâle et la partie femelle (3, 4) pouvant intervenir pour provoquer un déplacement de l'obturateur (5) ainsi que du corps mâle (3a) et de la douille (3d) vers leurs positions d'ouverture de la valve dans laquelle un trajet pour le liquide est établi entre la partie mâle et la partie femelle par l'intermédiaire de l'obturateur (5) et de l'orifice (3c), et le mouvement de débranchement entre la partie mâle et la partie femelle pouvant intervenir pour provoquer un déplacement de l'obturateur (5) et un déplacement du corps mâle (3a) et de la douille (3d) vers leurs positions d'ouverture de la valve, la douille (3d) comprenant un premier moyen de blocage (3f) pouvant coopérer, dans la position d'ouverture de la valve coulissante, avec un second moyen de blocage (4h) associé au corps femelle (4c) pour bloquer la douille de manière détachable en vue de l'empêcher de se séparer de la partie femelle pendant le mouvement de débranchement initial du corps mâle de manière à amener la douille à coulisser par rapport au corps mâle vers sa position de fermeture de la valve, le corps mâle, la douille, le corps femelle et l'obturateur coopérant les uns avec les autres, pendant le mouvement de débranchement, d'une manière qui évite efficacement la formation entre eux de vides quelconques à même de recevoir suffisamment de liquide pour former des gouttes, caractérisé en ce que la douille (3d) est faite d'une matière plastique synthétique, le premier moyen de blocage (3f) comprend un moyen élastique faisant partie intégrante de la douille et pouvant coopérer avec le second moyen de blocage (4h) pour bloquer la douille (3d) de manière élastiquement détachable en vue de l'empêcher de. se séparer de la partie femelle pendant le mouvement de débranchement initial et l'obturateur (5) comprend une face généralement transversale pouvant coopérer avec le siège de valve (4e).

2. Raccord suivant la revendication 1, caractérisé en ce que l'extrémité fermée du corps mâle (3a) est fermée par une paroi d'extrémité transversale extérieurement plane (3b) et l'obturateur (5) comprend un obturateur en forme de disque qui, dans la position de fermeture de la valve, présente une face transversale généralement plane tournée vers l'extrémité ouverte du corps femelle (4c) recevant l'extrémité ouverte du corps mâle et pouvant coopérer de manière étanche avec le siège de valve (4e), le siège de valve présentant un orifice calibré pour recevoir étroitement la paroi d'extrémité (3b) de sorte que, dans la position de fermeture de la valve, au niveau de la transition entre la position d'ouverture de la valve et la position de fermeture, la paroi d'extrémité (3b) coopère étroitement avec l'obturateur en forme de disque (5) et avec l'orifice du siège de valve (4e) pour empêcher la formation entre eux de vides significatifs quelconques à même de recevoir du liquide.

3. Raccord suivant la revendication 1 ou 2, caractérisé en ce que le premier moyen de blocage (3f) comprend une bride élastique faisant partie intégrante de l'extérieur de la douille (3d) à l'extrémité de celle-ci éloignée de l'orifice prévu dans le corps mâle, la bride faisant saillie vers le corps femelle et pouvant coopérer avec le second moyen de blocage (4h) qui comprend un épaulement dans le corps femelle (4c).

4. Raccord suivant la revendication 3, caractérisé en ce que le corps mâle (3a), le corps femelle (4c) et la douille (3d) sont généralement cylindriques, la bride (3f) est une bride annulaire et l'épaulement (4h) est défini par un évidement annulaire dans le corps femelle.

5. Raccord suivant la revendication 1, 2 ou 3, caractérisé en ce que l'introduction de la partie mâle dans la partie femelle pendant le mouvement de branchement sollicite la douille (3d) en contact avec le siège de valve (4e) de manière à déterminer le degré maximum d'introduction de la douille dans le corps femelle et à assurer que le corps mâle soit avancé à travers l'orifice prévu dans le siège de valve par rapport à la douille jusqu'à une position dans laquelle la douille n'obture pas l'ouverture (3c) du corps mâle, dans laquelle la paroi d'extrémité (3b) coopère avec l'obturateur (5) pour le dégager du siège de valve (4e) et dans laquelle l'ouverture (3c) dans le corps mâle soit disposé du côté du siège de valve (4e) éloigné de la douille.

6. Raccord suivant l'une quelconque des revendications précédentes, caractérisé en ce que, pendant le mouvement de branchement initial, la douille (3d) agrippe le corps mâle (3a) par friction avec une force qui est supérieure à celle avec laquelle le corps femelle agrippe la douille, la douille restant dans une position sur le corps mâle dans laquelle elle obture l'ouverture (3c) dans le corps mâle pendant le mouvement de branchement initial.

7. Raccord suivant l'une quelconque des revendications précédentes, caractérisé en ce que la section interne du corps femelle est réduite dans la région (4g) du siège de valve (4e) de manière à augmenter l'agrippage par friction du corps femelle sur la douille dans cette région (4g).

8. Raccord suivant la revendication 2 ou l'une quelconque des revendications 3 à 7, en combinaison avec la revendication 2, caractérisé en ce que la paroi d'extrémité transversale généralement plane (3b) du corps mâle est disposée d'une manière générale dans le plan de l'extrémité de la douille (3d) dans la position de fermeture de la valve.

9. Système d'écoulement de liquide chirurgical ou appareil d'écoulement de liquide comprenant un ou plusieurs raccords à jeter après usage suivant l'une quelconque des revendications précédentes, et comportant un système ou un appareil de drainage chirurgical comprenant un récipient à liquide (1), un cathéter ou un autre moyen de drainage pouvant coopérer avec un patient et un tuyau de liquide (2) raccordant le drain et le récipient et comprenant le raccord.

10. Raccord, système ou appareil suivant l'une quelconque des revendications précédentes, comprenant au moins une valve de retenue (2c ou 2d) pour empêcher un écoulement dans un sens à travers le raccord lorsqu'il se trouve dans la position d'ouverture de la valve et la partie femelle (4) comprend un corps flexible (4b) contenant la seconde valve (5), le corps (4b) pouvant être comprimé à la main pour forcer du liquide à traverser le raccord lorsqu'il se trouve dans la position d'ouverture de la valve.

Fig.1

Fig.2